# EUROPEAN PATENT APPLICATION

(11) **EP 3 666 303 A1**
(43) Date of publication of application: **17.06.2020**
(21) Application number: 18211647.5
(22) Date of filing: 11.12.2018
(51) Int. Cl.: A61M 1/12, A61M 1/10

(54) **CATHETER FOR INTRAVASCULAR BLOOD PUMP**

(71) Applicant: Abiomed Europe GmbH, 52074 Aachen (DE)
(72) Inventor: Mourran, Claudia, 52074 aachen (DE)
(74) Representative: Klunker IP Patentanwälte PartG mbB

(57) **Abstract**

An intravascular blood pump (P) comprises a catheter (5) and a pumping device (1) attached to a distal end (15) of the catheter (5). The blood pump (P) is advanced through a patient's blood vessel by means of the catheter (5). The catheter (5) has an elongate tubular body (10) and a porous three-dimensional structure (6) provided on at least a portion of the outer surface (8) of the catheter body (10) to promote adsorption of proteins and formation of an autologous graft (7) to prevent the catheter (5) from growing into the inner wall of the blood vessel. The porous three-dimensional structure (6) may be formed as a textile sleeve (6), preferably made of a warp knitted fabric.

## Description

### FIELD OF THE INVENTION

This invention relates to an intravascular blood pump for percutaneous insertion into a patient's blood vessel, in particular to be advanced into the patient's heart, and more specifically to a catheter of the intravascular blood pump which reduces or prevents ingrowth into tissue of the blood vessel.

### BACKGROUND OF THE INVENTION

An intravascular blood pump, which is designed to be inserted percutaneously into a patient's blood vessel, such as a femoral or axillary artery or vein, may be advanced into the patient's heart to act as a left ventricular assist device or right ventricular assist device. The blood pump may, thus, be referred to also an intracardiac blood pump. An intravascular blood pump typically comprises a catheter and a pumping device attached to a distal end of the catheter. The catheter may have an elongate tubular body and may contain supply lines, such as an electric line and a purge line. The pumping device may comprise an impeller which rotates during operation of the blood pump to convey blood from a blood flow inlet to a blood flow outlet of the blood pump, for instance through a flow cannula. Throughout this disclosure, the term "distal" will refer to directions away from a user and towards the heart, whereas the term "proximal" will refer to directions towards a user.

Intravascular blood pumps may be applied in short-term applications for a few hours or days or in long-term applications for weeks or even months. Typically, an intravascular blood pump may be used as a bridge to recovery, which means that the blood pump is removed from the patient after the heart has reached a sufficient recovery and no longer needs to be supported by the blood pump. However, particularly in long-term applications, removal of the blood pump may be difficult because it may be encapsulated by tissue of the blood vessel. Encapsulation of foreign objects by tissue overgrowth and possibly ingrowth is a natural reaction of the human's body against foreign objects to form a protection barrier between the body and the foreign object inside body. Generally, the material and surface of the foreign object, its geometry, mechanical irritation caused by the object and the like trigger tissue encapsulation of the foreign object.

With respect to an intravascular blood pump, particularly the catheter, which extends through a blood vessel towards the heart, may be treated as a foreign object by the patient's body and may thus be subject to tissue ingrowth or overgrowth due to its proximity to the vessel wall. In particular, mechanical irritations of the inner vessel wall caused by contact with the catheter may promote tissue ingrowth. Tissue ingrowth is a relevant problem in long-term applications, for instance 30 days or longer operation of the blood pump. High forces (e.g. up to 50 N) may be necessary for removal of the blood pump, which would cause severe trauma and injuries to the blood vessel and can lead to particulate emissions, which in turn may cause infarcts.

Different attempts have been made to reduce or avoid ingrowth of the catheter in the blood vessel. However, tests are difficult because apparently different species react differently with regards to tissue ingrowth or overgrowth. Tissue ingrowth or overgrowth starts on the interior of the vessel lumen and on the implant side with adsorption of fibrinogen on the outer surface of the catheter. After that, platelets (thrombocytes) and red blood cells (RBCs, erythrocytes) adhere to the fibrinogen layer. Finally, a thin sleeve containing fibrin forms on the outer surface of the catheter.

In order to avoid creation of such sleeve, catheters usually are provided with smooth surfaces, e.g. formed of extruded polyurethane (PU). However, creation of such sleeve will still take place, which may adhere to the vessel wall or may crumple on the smooth polyurethane surface. If the sleeve loosens, it may at least partially tear off such that an infarct may be caused. In order to avoid adsorption of fibrinogen, organic or inorganic (e.g. Ag) additives may be added to the polyurethane. Likewise, antithrombogenic, hydrophilic or hydrophobic coatings may be applied. Nevertheless, tissue ingrowth is still a problem and is a critical barrier for long-term applications for intravascular blood pumps.

### SUMMARY OF THE INVENTION

It is therefore an object of the present invention to provide an intravascular blood pump, more specifically a catheter for an intravascular blood pump, which allows easy removal of the blood pump from the patient after a certain amount of time, in particular in long-term applications, by reducing or avoiding tissue ingrowth.

This object is achieved according to the present invention by a catheter having the features of the independent claims. Preferred embodiments and further developments of the invention are specified in the claims dependent thereon.

According to an aspect of the invention, a catheter for an intravascular blood pump for percutaneous insertion into a patient's blood vessel is provided. According to another aspect an intravascular blood pump for percutaneous insertion into a patient's blood vessel is provided which comprises a pumping device and the catheter. The catheter of both aspects has an elongate tubular body which extends between a proximal end and a distal end and has an outer surface. According to the invention, the catheter includes a porous three-dimensional structure on at least a portion of the outer surface. The structure is porous, i.e. it has a plurality of apertures, which allow adsorption of blood proteins and other cells. The structure is three-dimensional, i.e. it particularly has a minimum dimension (thickness) in a radial direction as will be explained in more detail below.

Surprisingly, the porous three-dimensional structure is capable of reducing or preventing tissue ingrowth. While concepts with porous structures were tested with the intention to promote tissue ingrowth in order to secure the position of the catheter within a blood vessel, it has been found out that the porous structure reduces or even prevents tissue ingrowth. More specifically, according to the invention it is desired that the porous three-dimensional structure promotes creation of an autologous graft (referred to as "autograft"), which forms a slippery surface and prevents overgrowth or ingrowth of tissue, in particular tissue of an inner wall of a blood vessel in which the catheter is located during operation of the blood pump. In other words, the patient's body creates its own coating over the catheter in the region of the porous structure, i.e. an autologous coating.

The basis of the autograft may be adsorption of proteins, in particular fibrinogen. Thus, more generally and in contrast to known catheters, which are designed to avoid tissue ingrowth, a catheter for an intravascular blood pump is provided, having on at least a portion of its outer surface a porous three-dimensional structure, which is able to adsorb proteins, e.g. fibrinogen and/or other proteins. After adsorption of fibrinogen, platelets and red blood cells may adhere to the fibrinogen layer. Further cells, e.g. myointimal cells and/or fibrinogen adhere to form a stable, homogenous autograft. Consequently, as already mentioned above, after formation of the autograft, which is an autologous coating, the autograft overlies at least the respective portion of the outer surface of the catheter which supports the porous three-dimensional structure, such that an outer surface of the autograft forms the outermost surface of the total assembly, comprising the catheter, the porous three-dimensional structure and the autograft.

The autograft is supported and held in place by the porous three-dimensional structure. It is slippery to allow the catheter to slide within the blood vessel without injuring or adhering to the inner vessel wall. The autograft formed on the porous structure reduces tissue irritation and thereby the natural reaction of the body to encapsulate foreign materials, and prevents the catheter from being overgrown by tissue such that the catheter can be easily removed even in long-term applications, e.g. of about three to six months, without causing trauma to the blood vessel. Even if trauma was caused in the blood vessel by initial mechanical irritation during insertion and advancement of the catheter, these injuries may heal with the catheter in place. The porous structure may be located along the entire length of the catheter or only on portions of the catheter body which are placed in larger vessels during operation of the blood pump to avoid mechanical irritation of the vessel or ingrowth.

In a preferred embodiment, the porous three-dimensional structure is formed by a sleeve arranged on the outer surface of the catheter body. The sleeve may comprise or may be formed of a textile material. In particular, the sleeve may comprise a knitted fabric, preferably formed by warp knitting. Suitable warp knitting techniques may be used, such as 1x1 constructed knits (also known as tricot) or 2x1 constructed knits. Such warp knitted fabrics have good elasticity properties and are able to follow the movements and bends of the catheter without significantly affecting the mechanical characteristics of the catheter body. Furthermore, the mesh structure of a textile material, in particular a knitted fabric, provides a support structure with apertures and webs for adhesion of proteins and cells to form the aforementioned autograft.

The knitted fabric may include multi-filaments, each multi-filament preferably comprising 3 to 100 filaments, preferably 15 to 30 filaments, more preferably 24 filaments. The textile material of the sleeve may be produced by melt spinning process, which allows for creation of very thin filaments, e.g. having a diameter of about 1 µm to 100 µm, preferably 2 µm to 30 µm and more preferably 10 µm to 20 µm.

With regards to textile materials, e.g. for warp knitted fabrics, the linear mass density of fibers, yarns and thread and is defined as the mass in grams per 1000 meters and measured in the unit "tex". One example of PET fabric with 24 filaments per multi-filament may be PET 78dtex/24f. Other examples of suitable textile materials for the sleeve may be polyamide in the range of 17dtex/3f to 110dtex/34f, or PES in the range of 33dtex/24f to 180dtex/88f.

Further to the above-mentioned example, multi filaments with very thin filaments e.g. 0.5 µm to 10 µm, preferably 1.7 µm to 5 µm and more preferably 2 µm to 4 µm may be used. Those filaments can be obtained by the so-called "island-in-the-sea"-process, for example. The number of filaments per thread has to be adapted according to the desired thread diameter.

The sleeve may have an elongate tubular body having a proximal end and a distal end. The sleeve may be attached to the tubular body of the catheter at least at the sleeve's proximal end and the sleeve's distal end. It will be appreciated that the sleeve may be attached to the catheter body also at further locations along the length of the sleeve's body or along the entire length of the sleeve. However, if the sleeve is secured only at its ends, the mechanical properties of the catheter substantially will not be affected. Advantageously, the sleeve is attached to the catheter body in a glue-free manner, i.e. without using an additional adhesive, which could fail during applications. For instance, the sleeve may be attached to the catheter body by solvent welding, where a solvent such as tetrahydrofuran (THF), dimethyl sulfoxide (DMSO), dimethylformamide (DMF) or the like is used to etch the catheter body while not affecting the sleeve material. The sleeve then firmly bonds with the catheter body.

The sleeve is advantageously tightly fitted on the outer surface of the tubular body. In other words, the sleeve may have an inner diameter substantially equal to or only slightly larger than the outer diameter of the catheter body. A tight fit of the sleeve over the catheter body may be preferable compared to a loose fit in order to avoid tissue or clots to accumulate inside the sleeve, i.e. between the sleeve and the catheter body. In case of a tight fit, the sleeve may be stiffer in a radial direction compared to an axial direction. In particular, the sleeve may be axially compliant, in particular with respect to axial extension and axial compression. More specifically, the sleeve may be axially extendable and axially compressible, which allows the sleeve to sit snuggly tight on the catheter while permitting the catheter to bend without causing wrinkles.

However, if desired, the sleeve may be fitted in a loose manner, i.e. may have an inner diameter larger than the outer diameter of the catheter body. If the sleeve is fitted in a loose manner such that a clearance may exist between the outer surface of the catheter body and an inner surface of the sleeve, the autograft may at least partially encapsulate the sleeve, i.e. the autograft may extend radially through the sleeve or from both a radially outer surface of the sleeve and the radially inner surface of the sleeve, and may thus contact the outer surface of the tubular body of the catheter.

Preferably, the porous structure comprises or is made of a non-absorbable material. The porous structure serves as a support structure or scaffold for the autograft. Thus, it is preferable if the porous structure is stable over time and during operation of the blood pump and does not resorb. Independent from its resorption characteristics, the porous structure may comprise a radiopaque material. This facilitates observation of the correct placement of the catheter, in particular correct placement of the porous structure. More specifically, it can be observed whether the porous structure, which may extend only along a portion of the length of the catheter, is placed correctly in critical areas of the blood vessels in which tissue overgrowth is most likely to occur. For instance, some of the threads of a textile material may comprise a radiopaque material to act as radiopaque markers.

Suitable materials for the porous structure may be polyethylene (PE), polypropylene (PP), polyamide (PA), polyether sulfone (PES), polyethylene terephthalate (PET) and polyurethane (PU). The textile material of the sleeve may comprise filaments made of one or more of the aforementioned materials. The sleeve may be made of one material or a combination of materials. For instance, the warp and weft of a textile material may be made of different materials.

The porous structure may define a three-dimensional structure configured to promote adsorption of protein and cells. More specifically, the three-dimensional structure may include a plurality of apertures (or openings) and a plurality of webs (or struts) permitting formation of protein and cells in a radial inward direction. The apertures and webs may be formed by the apertures and threads of a textile material or other mesh or net structure. Alternatively, the porous three-dimensional structure may be formed from a foam or sponge-like structure or any other regular or irregular structure with pores which is able to promote adsorption of protein and cells. If desired, the porous structure may be integrally formed with the tubular body of the catheter on the outer surface thereof, or may be separately formed, e.g. in the form of the aforementioned sleeve, or may be manufactured directly onto the catheter, e.g. by electrospinning.

The porous three-dimensional structure may comprise a single or more than one layer of one or more of the aforementioned configurations, such as a textile sleeve, mesh, foam or other porous structure. The individual layers may be formed of the same or different configurations. For instance, a foam-like or sponge-like structure may be combined with a textile sleeve surrounding the foam or sponge.

In an embodiment, the porous structure may define a plurality of first apertures and a plurality of second apertures, the first and second apertures being different in size. In other words, in case of a textile material the mesh size may vary. This may improve formation of the aforementioned autograft. With regards to a porous structure having more than one layer as described above, the aperture sizes may vary or may be the same in the individual layers to permit different cells to adhere in the different layers of the three-dimensional porous structure.

The porous structure, in particular the aforementioned three-dimensional structure, more specifically the aforementioned sleeve, may have a thickness of at least 20 µm, preferably at least 30 µm. The thickness of the porous three-dimensional structure is measured in a radial direction extending from the outer surface of the tubular body of the catheter to the outer surface of the porous structure. In case a clearance exists between the porous structure and the outer surface of the catheter body, the thickness of the porous structure may be measured from the inner surface of the porous structure to the outer surface of the porous structure in a radial direction. In other words, the wall thickness of the sleeve should have a minimum extension to improve the function of the sleeve as support structure for the autograft. In particular, a minimum radial thickness allows the autograft to grow in a radial inward direction from an outer surface of the porous structure such that the overall diameter substantially does not increase after initial formation of the autograft. It will be appreciated that the overall diameter of the catheter may be 12 F (4 mm) or less, preferably 9F (3 mm) or less, possibly 6F (2 mm) or 4F (1.3 mm), to avoid irritation or injury of the vessel wall. If the vessel size is too small relative to the implanted catheter, the caused mechanical irritation could again lead to overgrowth of tissue despite the presence of the autograft.

### BRIEF DESCRIPTION OF THE DRAWINGS

The foregoing summary, as well as the following detailed description of preferred embodiments, will be better understood when read in conjunction with the appended drawings. For the purpose of illustrating the present disclosure, reference is made to the drawings. The scope of the disclosure is not limited, however, to the specific embodiments disclosed in the drawings. In the drawings:
Figs. 1a and 1b schematically show an intravascular blood pump inserted into a patient's heart via different types of access.
Fig. 2 shows a cross-section of the catheter of the blood pump of Fig. 1 with the sleeve.
Fig. 3 shows an embodiment of a knit pattern for the sleeve.
Fig. 4 shows another embodiment of a knit pattern for the sleeve.
Fig. 5 shows an alternative embodiment for a porous three-dimensional structure.
Fig. 6 shows a further alternative embodiment for a porous three-dimensional structure.

### DETAILED DESCRIPTION

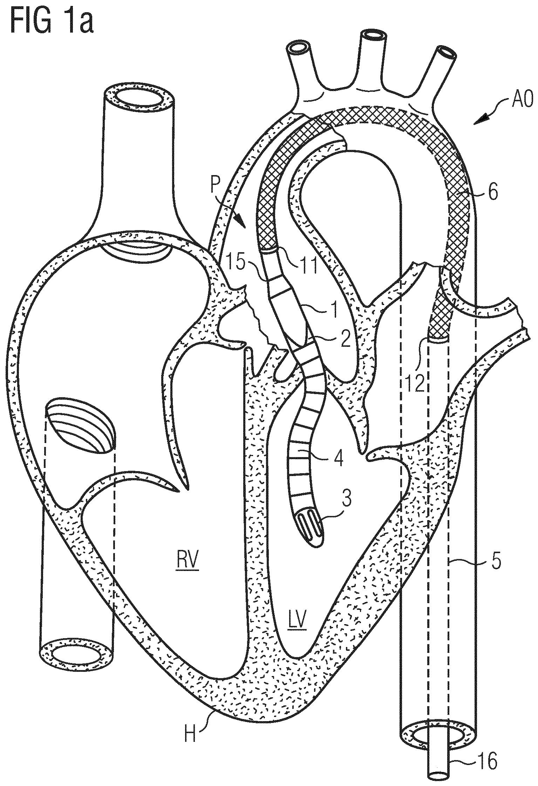

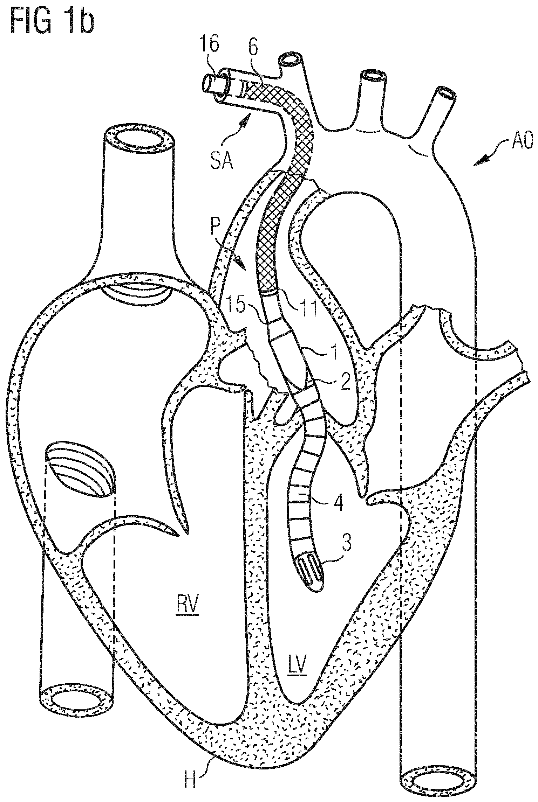

In Figs. 1a and 1b is illustrated an intravascular blood pump P inserted into a patient's heart H. More specifically, the blood pump P comprises a pumping device 1 attached to a catheter 5 by means of which the pumping device 1 is inserted into the left ventricle LV of the patient's heart H to pump blood from the left ventricle LV into the aorta AO. The shown application is only an exemplary application, and the blood pump P of the present invention is not limited to this application. For instance, reverse applications for the right ventricle RV may be envisioned. The blood pump P is percutaneously inserted e.g. via a femoral access and is advanced through the aorta AO into the heart H (see Fig. 1a). Alternatively, the blood pump P may be percutaneously inserted via an axillary access and advanced via a subclavian artery SA and through the aorta AO into the heart H (see Fig. 1b). The blood pump P is placed such that a blood flow outlet 2 is disposed outside the patient's heart H in the aorta AO, while a blood flow inlet 3 which is in flow communication with a flow cannula 4 is disposed inside the left ventricle LV. An impeller (not shown) is provided in the pumping device 1 to cause the blood flow from the blood flow inlet 3 to the blood flow outlet 2, and rotation of the impeller is caused by an electric motor (not shown) disposed in the pumping device 1.

The intravascular blood pump P is advanced into the patient's heart by means of the catheter 5, wherein the pumping device 1 is attached to a distal end 15 of the catheter 5 opposite a proximal end 16 of the catheter 5. As schematically illustrated in Fig. 1, the catheter 5 may contact the inner wall of the aorta AO, either during insertion or during operation or most likely both. This may cause irritation or injury of the vessel (triggered by the surface topology and a foreign material) wall and prompts overgrowth of the catheter 5 with bodily tissue. Proteins and other cells contained in the blood will adhere to the outer surface of the catheter 5. In particular, initially proteins and cells will adhere to the outer surface of the catheter 5. Therefore, known catheters tend to grow in the tissue of the vessel, which makes it difficult to remove the blood pump P from the patient's body. This will occur particularly in long-term application, where the blood pump P is in operation for several weeks or months. Removal of the ingrown catheter may cause injuries to the blood vessel.

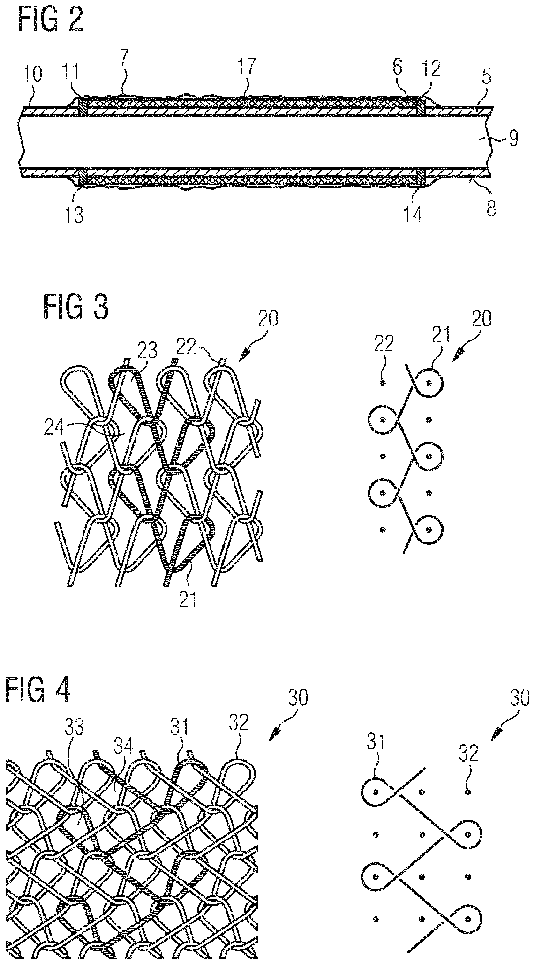

In order to reduce or avoid tissue ingrowth or overgrowth, a porous three-dimensional structure in the form of a textile sleeve 6 is provided along at least a portion of the catheter 5, in particular in a portion which tends to contact the inner vessel wall. Although the sleeve 6 induces adhesion of fibrinogen and could be considered to promote tissue ingrowth, it has been found that the opposite occurs due to formation of an autograft 7 (see Fig. 2) on the sleeve 6, which slides along the inner vessel wall as will be explained in more detail below.

Referring now to Fig. 2, a cross-section of the catheter 5 with the sleeve 6 mounted thereon is schematically illustrated. The catheter 5 has a tubular body 10 with an outer surface 8 and a lumen 9. The sleeve 6 has a tubular body 17 with a distal end 11 and a proximal end 12 and is attached to the body 10 of the catheter 5 by a suitable attachment technique, in particular solvent welding, which does not require the use of additional adhesive. As indicated in Fig. 2 at reference numerals 13 and 14, the sleeve 6 is attached to the catheter body 10 only at its distal end 11 and proximal end 12. Thus, the major part of the sleeve 6 between its ends 11, 12 is not attached to the outer surface 8 of the catheter body 10, such that the mechanical characteristics of the catheter 5 are substantially not affected by the sleeve 6. Further, it will be appreciated, while the sleeve 6 is shown to be formed of a single layer, it may comprise more than one layer. The layers may be identical or different, e.g. with respect to the size of the apertures formed by the knitted fabric described below.

The textile sleeve 6 may be made of a warp knitted fabric. A warp knitted fabric has good elasticity properties and provides a support structure with apertures to promote adsorption of the autograft 7. Other textile materials, such as knotted fabrics, woven fabrics or non-woven materials may be used if they are suitable for inducing formation and for supporting the autograft. Examples of known warp knitted fabrics, which have been found to be particularly suitable for the sleeve 6, are schematically shown in Figs. 3 and 4. Fig. 3 shows a knitted fabric 20 as 1x1 constructed knits (also known as tricot). Fig. 4 shows a knitted fabric 30 as 2x1 constructed knits. In order to visualize the knitting patterns, a distinct thread 21 is marked in Fig. 3 among the threads 22. Apertures 23, 24 of different size are formed between the threads 21, 22 to promote adsorption of fibrinogen and further adhesion of blood cells to form the autograft 7. The same applies for the threads 31, 32 and the apertures 33, 34 of the knitted fabric 30 shown in Fig. 4. Preferably, the threads 21, 22, 31, 32 are formed by multi-filaments to enhance the three-dimensional structure of the sleeve 6.

The autograft 7 grows into the three-dimensional structure provided by the sleeve 6, in particular the warp knitted fabrics 20, 30 comprising multi-filaments as explained above. Since the autograft 7 is stably supported by the sleeve 6, it does not crumble or loosen but provides a slippery autologous coating which prevents adhesion of the catheter 5 to the inner vessel wall. The autograft 7 grows into the sleeve 6, such that the overall diameter of the catheter 5 substantially does not increase after initial formation of the autograft 7. The autograft 7 covers the sleeve 6 such that the catheter 5 will not be encapsulated in the region of the sleeve 6 as a foreign object and can be easily removed without causing trauma to the blood vessel. Furthermore, if trauma is caused initially upon insertion of the blood pump P into the patient, healing may start even during operation of the blood pump P with the catheter 5 in place in the blood vessel.

Thus, the intentional and desired adsorption of proteins and other cells on the sleeve 6 does not lead to ingrowth of the catheter 5 into the vessel wall, but has the unexpected effect that an autologous coating is formed which allows the catheter to slide freely inside the blood vessel and prevents ingrowth of the catheter 5. The unexpected effect can be described by the different dynamics. That means, while the formation of the autograft starts immediately, as soon as the porous structure is immersed in blood, the catheter overgrowth takes weeks. Thus, as soon as the autograft is present (typically within days), the stimulus for overgrowth from the vessel wall onto the adjacent catheter is stopped and overgrowth does not occur.

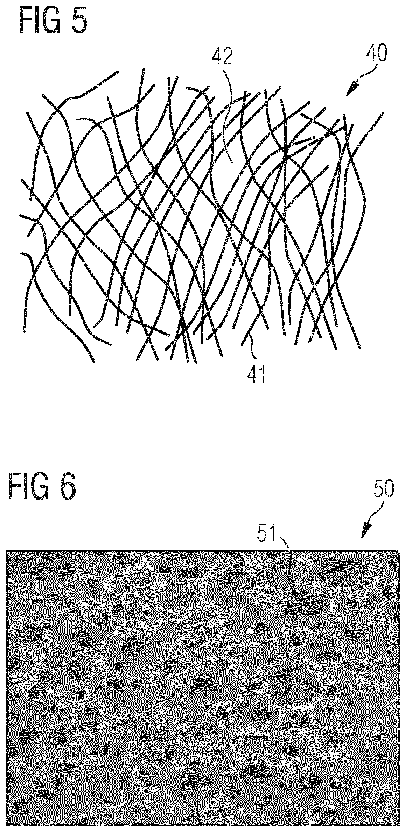

Figs. 5 and 6 schematically show further alternative embodiments for a porous three-dimensional structure which may be applied to a catheter instead of or possibly in addition to the aforementioned sleeve 6. Formation of the autograft occurs in the same way as explained above for the sleeve 6. Referring to Fig. 5, the porous three-dimensional structure 40 may be formed by electrospinning in one or more layers and may comprise a plurality of filaments 41 to form apertures 42 to permit formation of the autograft 7 as explained above. The filaments 41 and thus the apertures 42 may be arranged irregularly in desired dimensions. Electrospinning allows for manufacturing of the porous structure 40 directly onto the catheter 5. Likewise, a foam-like or sponge-like structure 50 having apertures 51 as shown in Fig. 6 may be formed directly on the catheter 5.

## Claims

1. A catheter (5) for an intravascular blood pump (P) for percutaneous insertion into a patient's blood vessel, the catheter (5) having an elongate tubular body (10) which extends between a proximal end (16) and a distal end (15) and has an outer surface (8), the catheter (5) including a porous three-dimensional structure (6) on at least a portion of the outer surface (8).

2. The catheter of claim 1, wherein the porous three-dimensional structure (6) is formed by a sleeve (6) arranged on the outer surface (8) of the tubular body (10).

3. The catheter of claim 2, wherein the sleeve (6) comprises a textile material.

4. The catheter of claim 2 or 3, wherein the sleeve (6) comprises at least one of a knitted fabric, a knotted fabric, a woven fabric or a nonwoven.

5. The catheter of claim 3 or 4, wherein the sleeve (6) comprises a knitted fabric (20; 30) formed by warp knitting, preferably formed as 1x1 constructed knits (20) or 2x1 constructed knits (30).

6. The catheter of any one of claims 2 to 4, wherein the sleeve (6) comprises a knitted fabric (20; 30) including multi-filaments, each multi-filament preferably comprising 3 to 100 filaments, preferably 15 to 30 filaments, more preferably 24 filaments.

7. The catheter of any one of claims 2 to 5, wherein the sleeve (6) has an elongate tubular body (17) having a proximal end (12) and a distal end (11) and is attached to the tubular body (10) of the catheter (5) at least at the sleeve's proximal end (12) and the sleeve's distal end (11), preferably in a glue-free manner.

8. The catheter of any one of claims 2 to 6, wherein the sleeve (6) is tightly fitted on the outer surface (8) of the tubular body (10) of the catheter (5).

9. The catheter of any one of the preceding claims, wherein the porous three-dimensional structure (6) comprises a non-absorbable material.

10. The catheter of any one of the preceding claims, wherein the porous three-dimensional structure (6) comprises a radiopaque material.

11. The catheter of any one of the preceding claims, wherein the porous three-dimensional structure (6) comprises at least one of polyethylene, polypropylene, polyamide, polyether sulfone, and polyethylene terephthalate.

12. The catheter of any one of the preceding claims, wherein the porous three-dimensional structure (6) is configured to promote adsorption of fibrinogen, the three-dimensional structure preferably including a plurality of apertures (23, 24; 33, 34) and a plurality of webs (21, 22; 31, 32) permitting adsorption of fibrinogen in a radial inward direction.

13. The catheter of any one of the preceding claims, wherein the porous three-dimensional structure (6) has a thickness of at least 20 µm, preferably at least 30 µm.

14. A catheter (5) for an intravascular blood pump (P) for percutaneous insertion into a patient's blood vessel, the catheter (5) having an elongate tubular body (10) which extends between a proximal end (16) and a distal end (15) and has an outer surface (8), wherein at least a portion of the outer surface (8) is configured to promote adsorption of proteins and blood cells.

15. An intravascular blood pump (P) for percutaneous insertion into a patient's blood vessel, comprising a pumping device (1) and a catheter (5) of any one of the preceding claims.
